# EUROPEAN PATENT SPECIFICATION

(11) **EP 1 676 550 B1**
(45) Date of publication and mention of the grant of the patent: **08.08.2007**
(21) Application number: 04766954.4
(22) Date of filing: 22.09.2004
(51) Int. Cl.: A61F 5/08

(54) **NASAL STIMULATOR**
NASENSTIMULATOR
STIMULATEUR NASAL

(30) Priority: 22.09.2003 ES 200302195
(43) Date of publication of application: 05.07.2006
(73) Proprietor: Institute Orthodontic World J. Duran Von Arx, S.L., E-08031 Barcelona (ES)
(72) Inventor: DURAN VON ARX, Josep, E-08031 Barcelona (ES)
(74) Representative: Urizar Anasagasti, José Antonio
(86) International application number: PCT/ES2004/000416
(87) International publication number: WO 2005/027804

(56) References cited:
- FR-A- 2 613 232
- US-A- 2 569 743
- US-A- 2 672 138
- US-B1- 6 386 197
- US-B1- 6 564 800

## Description

### OBJECTIVE OF THE INVENTION

Nasal stimulator whose function is to dilate and stimulate the levator muscle of the alar sidewall of the nose to improve breathing for persons who suffer nasal blockage hence their breathing is done mainly through the mouth. This invention alleviates this problem by inserting a cylinder with interior perforations made of silicone into one or each nostril, and when a dilation is produced in the nostril, it eases breathing. The closest prior art is US-A-2672138.

### BACKGROUND OF THE INVENTION

We are all aware of the discomforts produced by incorrect breathing through the nose. These discomforts can be produced by different reasons, such as having a deviated septum, lack of stimulation of the nasal muscle, having defective nasal cartilage, etc.

There are many different products in the market which solve breathing problems that people with a defective nasal structure have. These are applied by inserting them in the nostrils either in liquid form or in spray, with the objective of causing the nasal muscle to dilate therefore, allowing normal breathing for a certain period of time.

The actual invention avoids the use of any type of liquid or spray in the nostrils, since these products are less effective in the long run and can cause a kind of addiction to them. On the contrary, the actual invention of the nasal stimulator acts by inserting in one or each nostril, depending on each individuals needs, an internally perforated silicone cylinder with a featured widened periphery except in the area which faces the nasal septum or wall which dilates the opening space or dome, stimulates the levator muscle of the nasal alar and helps the nasal wall or septum center remodel the nasal cartilage; the said cylinder also contains a protruding lip on its lower rim which has contact with the external part of the alar sidewall of the nose and stimulates the levator muscle of the noses' alar sidewall through the exterior. The summary of the anterior explanation is that the said stimulator facilitates the users breathing through the nose.

This present invention of the nasal stimulator is designed for use by all the persons with nasal breathing problems, which could include athletes and individuals with general orthodontic problems.

### GENERAL DESCRIPTION OF THE INVENTION

The invented nasal stimulator is defined in claim 1 and is composed of one or two silicone cylinders of approximately 1 cm in height which are perforated in their interior and which widen in the central part of the external surface. This widening is generally peripheral except in the area of the cylinder which faces the nasal septum. The grazing or touch that occurs between the said widening and the internal part of the nose stimulates the levator muscle of the nasal alar and the widening also causes a slight enlargement of the nasal orifice.

The lower part of each silicone cylinder ends in a lip or rim to impede the cylinder/s from going in the nostrils further than advisable when they are inserted in the nose to dilate the noses opening space or domes. A type of support that jutts out is integrated onto this rim which makes contact with the external part of the alar of the nose and stimulates the levator muscle of the nasal alar through the exterior side of the same, it adds pressure on the exterior of the users nose and allows the joining and fastening of the nasal stimulator.

The joint effect of the internal grazing of the widened part to the internal side of the nostril, and the external grazing or touch of the protruding support with the external part of the alar of the nose, and consequently the dilation of the nostrils facilitates therefore, the comfortable breathing of the patient.

### BRIEF DESCRIPTION OF THE DRAWINGS

For a better understanding of the objective of the said invention it is described as a practical realization of the invention around the basis of the attached figures where
Fig. 1 - shows a perspective view of the one cylinder embodiment of the nasal stimulator
Fig.2- shows a ground plan view of the one cylinder embodiment of the nasal stimulator,
Fig.3- shows a side elevation view of the one cylinder embodiment of the nasal stimulator.
Fig.4- shows a section as in plan E-E of Figure 3.
Fig.5- shows a perspective view of a double cylinder embodiment of the nasal stimulator
Fig. 6- shows a ground plan view of the nasal stimulator of Fig. 5.
Fig. 7- shows a side elevation view of the nasal stimulator of Fig. 5
Fig. 8shows a side view of the nasal stimulator of Fig.5

### PRINCIPAL EMBODIMENTS OF THE INVENTION

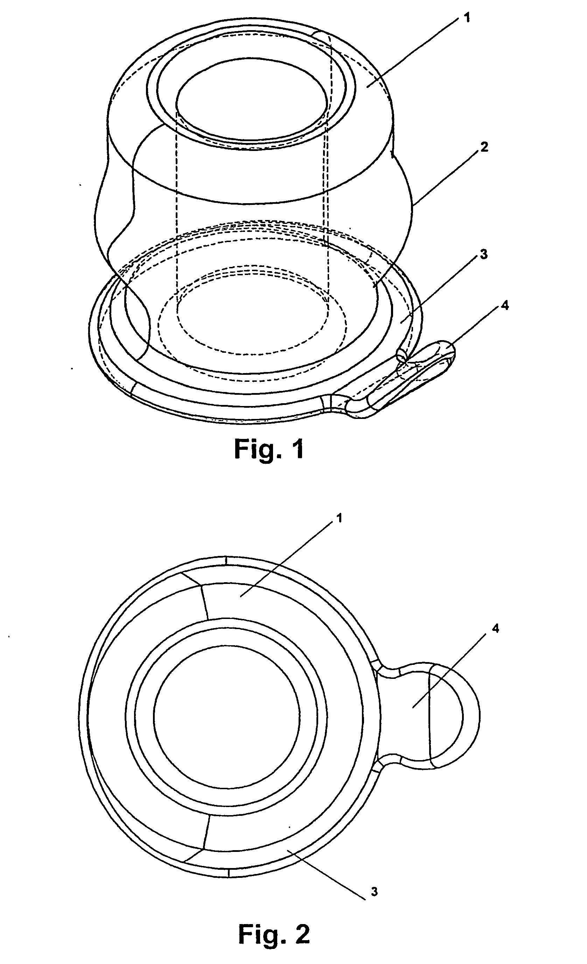

In an initial embodiment, the nasal stimulator invented is composed of, according to what is seen in Figs.1-4, a cylinder (1), perforated inside and made of silicone. Approximately halfway its exterior perimeter height, the cylinder widens in shape(2) throughout its whole periphery with the exception of the same area which faces the septum wall, whose function is to stimulate the levator muscle of the alar of the nose through the internal side of the nostril. This stimulation is caused by the touch or "grazing" that is produced between the widened part(2) and the interior of the nose which results in easing the respiration or breathing. In the lower part of the cylinder or the part that is left out when the user inserts the stimulator in the nose, the cylinder(1) has a peripheral rim(3) which serves as a limit where the insertion of the stimulator into the nose should not pass, integrated onto this rim(3) is a protruding support or lip(4) which adds pressure on the exterior of the users nose and allows for the coupling or joining and fastening of the nasal stimulator. Moreover, and of utmost importance, the contact and touch with the external alar of the nose stimulates the levator muscle of the alar through the exterior of the same.

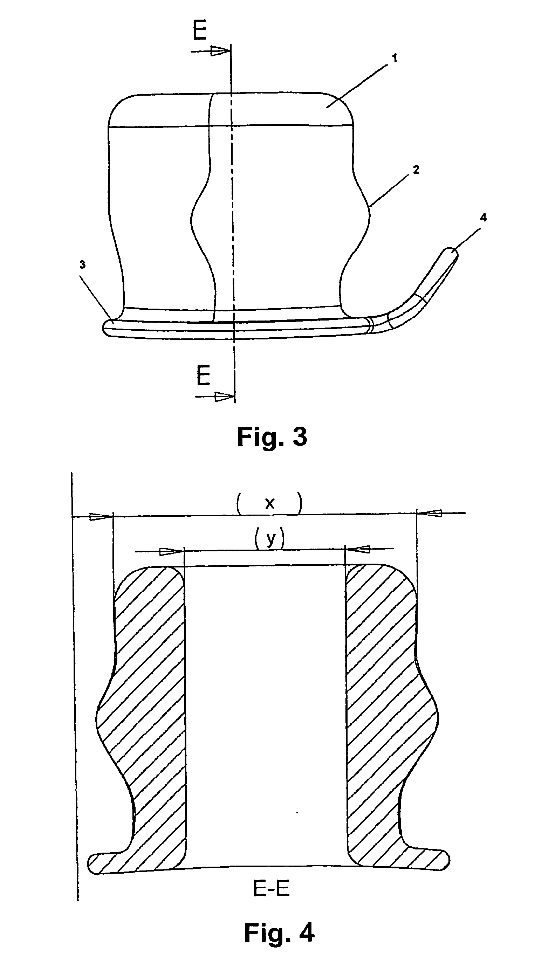

For a second embodiment, the nasal stimulator is composed of 2 identical and independent cylinders and one is inserted in each nostril. Although the dimensions are not limitative on the nature of the invention and should be in accordance with the users nose dimensions, some orientative dimensions are: it is of 1cm in height, 8mm in interior diameter((Y) see fig. 4) and between 12-16mm in exterior diameter((X) see fig.4)

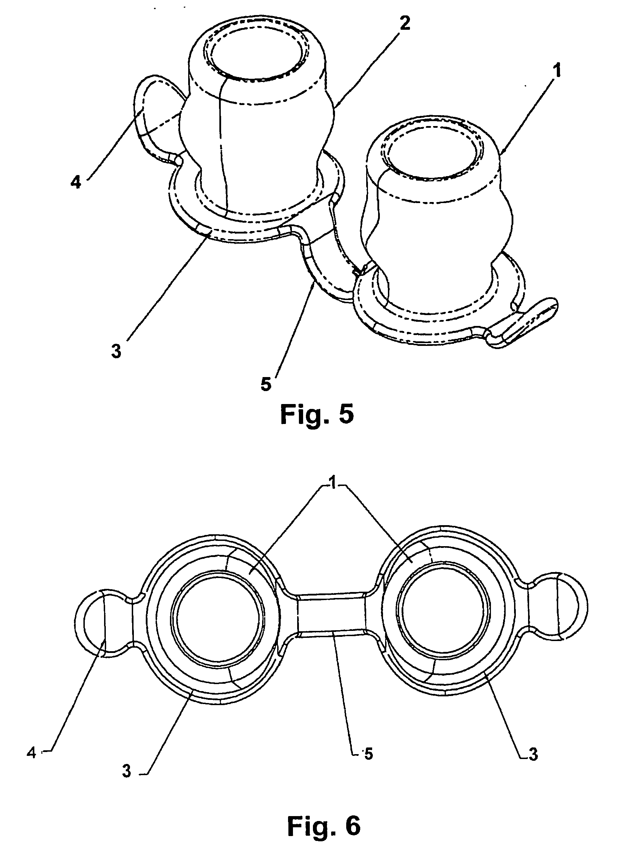

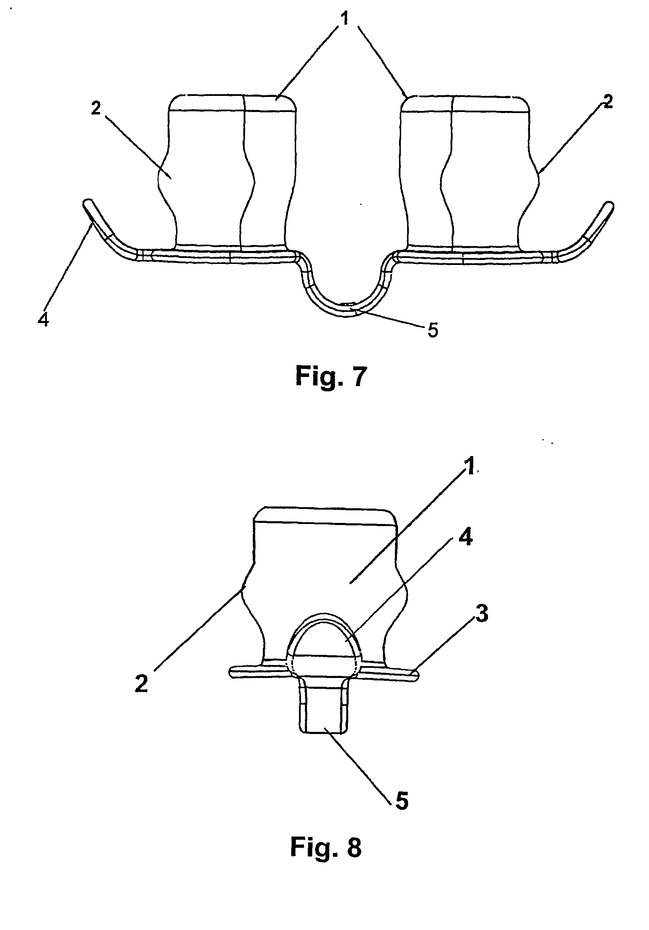

For a third embodiment, see fig.5-8, we can see the invented nasal stimulator formed by 2 cylinders(1), with their widenings (2) in the periphery of the cylinders except In the part of each which faces the nasal septum that is joined in the center by a tongue(5) that jutts out from the rims(3) in the part that is diametrically opposite the protruding supports(4) of each cylinder. The length of this tongue is enough so that one can insert both cylinders into the nostrils with the said tongue(5) slightly arched to facilitate its easy positioning.

Once the actual inventions nature is sufficiently described just as some main embodiments, it should be added that on its whole and on the parts that compose it, it is possible to introduce changes in shape, material and disposition, as long as the said alterations do not substantially vary the scope of the invention which is as claimed heretofore.

## Claims

1. Nasal stimulator consisting of one cylinder (1) or of two cylinders (1) joined by an curved tongue (5), whereby each cylinder is internally perforated and displays rim on its lower part (3) that keeps the cylinder from being inserted into the nostril deeper than it should, with the said rim (3) having a protruding support or tab (4), each cylinder (1) presenting a widening (2) in the central area of its external surface, said widening covering the whole periphery, except in a small area that will come to face the nasal septum during use, wherein said widening (2) produces a touch or grazing on the internal wall of the nose and said protruding support or tab (4) causes a touch or grazing on the external wall of the nose,
**characterised in that** both the widening (2) and the protruding support or tab (4) produce a gripping effect on the nose alar which stimulates the levator muscle of the nose, and that said nasal stimulator is made of silicone material.

## Patentansprüche

1. Nasenstimulator, bestehend aus einem Zylinder (1), oder aus zwei mittels einer gekrümmten Zunge (5) verbundenen Zylindern (1), wobei jeder Zylinder intern durchbohrt ist und auf seinem unteren Teil (3) ein Randwulst aufweist, welcher verhindert dass der Zylinder tiefer als nötig in das Nasenloch eingedrückt wird, wobei besagter Randwulst (3) eine vorragende Halterung oder Lasche (4), jeder Zylinder eine Verbreiterung (2) in der zentralen Zone seiner äusseren Oberfläche aufweist, wobei besagte Verbreiterung (2) den gesamten Aussenrand erfasst, ausser einer kleinen Zone die im Gebrauch dazu kommt der Nasenscheidewand gegenüberzustehen, wobei besagte Verbreiterung (2) eine Berührung oder Reibung der inneren Nasenwand erzeugt, besagte vorragende Halterung oder Lasche (4) eine Berührung oder Reibung der äusseren Nasenwand erzeugt, **dadurch gekennzeichnet dass** sowohl die Verbreiterung (2) wie auch die vorragende Halterung oder Lasche (4) eine klemmende Wirkung auf den Nasenflügel erzeugt, die den Nasenhebemuskel stimuliert, und dass besagter Nasenstimulator aus Silikon angefertigt ist.

## Revendications

1. Stimulateur nasal, qui comprend un cilindre (1) ou deux cilindres (1) unis par une languette (5) courbée, dans lequel chaque cilindre est perforé intérieurement et montre sur son côté inférieur (3) un bord qui empêche que le cylindre soit inséré plus profondément que nécessairement dans la narine, avec ledit bord (3) ayant un appui saillant ou rebord (4), chaque cylindre présentant un gonflement (2) dans la zone centrale de son surface externe, gonflement (2) couvrant la périphérie entière excepté dedans un petit secteur qui pendant l'utilisation arrive à faire face au septum nasal, où ledit gonflement (2) produit un contact ou frottement sur la parois interne du nez, ledit appui saillant ou rebord (4) cause un contact ou frottement sur le paroi externe du nez, **caracterisée en ce que** ledit gonflement (2) ainsi que ledit appui saillant ou rebord (4) produisent un effet de forcipressure sur l'aile du nez qui stimule le muscle élévateur du nez, et **en ce que** ledit stimulateur est fait du silicone.
